(19) Europäisches Patentamt
European Patent Office
Office européen des brevets

(11) Numéro de publication: **0 329 754 B1**

(12) **FASCICULE DE BREVET EUROPEEN**

(45) Date de publication de fascicule du brevet: **27.10.93** (51) Int. Cl.$^5$: **C12P 23/00**, C12M 1/00

(21) Numéro de dépôt: **88907711.1**

(22) Date de dépôt: **31.08.88**

(86) Numéro de dépôt internationale :
**PCT/FR88/00431**

(87) Numéro de publication internationale :
**WO 89/01977 (09.03.89 89/06)**

# (54) DISPOSITIF DE PRODUCTION DE CAROTENOIDES ET NOTAMMENT D'ASTAXANTHINE PAR CULTURE DE MICROALGUES.

(30) Priorité: **03.09.87 FR 8712250**

(43) Date de publication de la demande:
**30.08.89 Bulletin 89/35**

(45) Mention de la délivrance du brevet:
**27.10.93 Bulletin 93/43**

(84) Etats contractants désignés:
**CH GB IT LI**

(56) Documents cités:

**Chemical Abstracts, vol. 74, 1971 (Columbus, Ohio, US) F.C. Czygan: "Blood-rain and blood-snow: nitrogen, deficient cells of haematococcus pluvialis and chlamydomonas nivalis", voir p. 108, résumé 10715d**

(73) Titulaire: **COMMISSARIAT A L'ENERGIE ATOMIOUE**
**31/33, rue de la Fédération**
**F-75015 Paris Cédex 15(FR)**

(72) Inventeur: **GUDIN, Claude**
**Le Parc 4**
**Traverse Ste-Anne**
**F-13100 Aix-en-Provence(FR)**
Inventeur: **JUNGAS, Colette**
**Lotissement du Pont-de-l'Arc**
**Bâtiment B 5**
**F-13090 Aix-en-Provence(FR)**
Inventeur: **VAILLANT, Jean-François**
**11, rue Delaunoy**
**Bâtiment B.C.**
**F-77000 Melun(FR)**

(74) Mandataire: **Lhoste, Catherine et al**
**BREVATOME**
**25, rue de Ponthieu**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

Chemical Abstracts, vol. 96, 1983 (Columbus, Ohio, US), B. Renstroem et al.: "Natural occurence of enantiomeric and mesoastaxanthin. Part 3. Optical purity of (3,S,3'S)-astaxanthin from haematococcus pluvialis", voir p. 406, résumé 158845w

Chemistry and Industry, No. 23, 3 December 1984, (Londres, GB) S.J. Pirt: "Algal photosynthesis: the Aladdin's cave of biotechnology", pp. 843-849

## Description

La présente invention a pour objet un dispositif pour la production de caroténoïdes et plus spécialement d'astaxanthine par culture de microalgues. Elle s'applique avantageusement dans le domaine de l'agro-alimentaire et notamment en pisciculture pour la production de nourriture pour les poissons et les crustacés et en pharmacologie. En particulier, l'astaxanthine permet la pigmentation en rose de la chair des crevettes, des truites, des saumons, etc...

La coloration des différentes classes de microalgues est due aux trois principales catégories de pigments qui sont à l'origine des réactions photochimiques de la photosynthèse : les chlorophylles, les phycobilines et les caroténoïdes. Ces derniers sont liposolubles, isopréniques et issus de la voie de biosynthèse découlant de l'acide mévalonique. Ces pigments ou caroténoïdes sont de deux types, les carotènes et les xanthophylles.

Chaque espèce de microalgues contient de 5 à 10 caroténoïdes dont 1 ou 2 sont prépondérants.

Actuellement, on connaît une soixantaine de caroténoïdes différents chez les microalgues dont les plus communs sont le $\beta$ carotène, la fucoxanthine, la zéaxanthine, la dinoxanthine, la violaxanthine, l'anthéraxanthine, la myxoxantophylle, la lutéine, la pérydinine, la néoxanthine, la canthaxanthine, et l'astaxanthine.

Le $\beta$ carotène représente en particulier 10% en poids des microalgues Dunaliella bardwill et Dunaliella salina ; la lutéine peut être produite par Chlorella pyrenoïdosa à raison de 1 g/l de culture ; la diadinoxanthine représente en particulier 2,4% du poids de Vacularia virescens.

En ce qui concerne l'astaxanthine, pigment auquel s'applique plus particulièrement l'invention, de formule globale $C_{40}H_{52}O_4$, de poids moléculaire 596g et de formule développée :

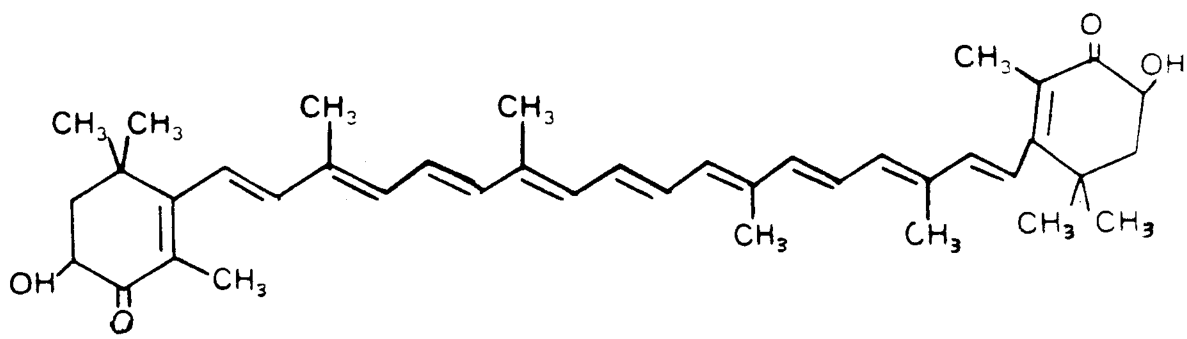

on peut citer comme microalgues contenant ce pigment Chlamydomonas nivalis, Euglena heliorobuescens et sanguinea, Balticola droebakensis, et Haematococcus Pluvialis connu aussi sous le nom de Haematococcus la custris. L'astaxanthine représente jusqu'à 1% en poids des trois premières microalgues et jusqu'à 5% en poids de la quatrième.

Pour d'autres renseignements concernant les microalgues et la synthèse des pigments par ces microalgues, on peut se référer à l'article de C. Gudin et C. Thepenier "Bioconversion of solar energy into organic chemicals by microalgue", paru dans Advances in Biotechnological Processes 6, pp. 73-110, 1986.

Par ailleurs, certaines levures renferment certains des pigments cités ci-dessus ; en particulier Pfaffia rhodozyma contient, jusqu'à 0,1% de son poids de matière sèche, d'astaxantine.

Les pigments cités précédemment peuvent être extraits des microalgues en contenant ou des animaux ayant mangé ces microalgues, ou bien être obtenus par synthèse chimique ; dans le cas particulier de l'astaxanthine, il est aussi possible de produire cette dernière par traitement des déchets de crevettes.

Les différents procédés actuellement connus de production de caroténoïdes, que cela soit par synthèse chimique ou traitement des microalgues contenant ces caroténoïdes, sont généralement longs et coûteux. En outre, leur rendement est faible.

La présente invention a justement pour objet un dispositif de production de caroténoïdes permettant de remédier à ces inconvénients. Il permet en particulier la production d'astaxanthine de façon simple et avec un rendement élevé.

Plutôt que d'extraire les caroténoïdes présents naturellement dans les microalgues, les inventeurs ont cherché à cultiver ces microalgues et à optimiser les paramètres jouant sur cette culture en vue d'augmenter la production de caroténoïdes par les microalgues correspondantes.

En particulier, les inventeurs ont trouvé que la production de caroténoïdes par Haematococcus pluvialis en présence d'au moins un composé azoté et d'au moins un composé carboné, peut être augmentée de façon importante si le rapport des concentrations C/N dans le milieu de culture, en fin de phase de

croissance de la microalgue, est au moins égal à 2 et au plus égal à 120.

Le rapport C/N peut être modifié soit en modifiant la quantité de carbone présente dans le milieu de culture, soit en modifiant la quantité d'azote présente dans le milieu de culture, soit en jouant simultanément sur la quantité de carbone et la quantité d'azote.

La quantité de caroténoïdes produite par Haematococcus pluvialis croît avec la quantité d'Haematococcus présente dans le milieu de culture. Aussi, la phase de croissance des microalgues doit être optimisée.

A cet effet, le rapport C/N en début de culture est choisi avantageusement entre 0,00018 et 0,3, la concentration en azote dissous allant de 40 à 120 mg/l et de préférence allant de 80 à 120 mg/l et la concentration en carbone dissous allant de 0,22 à 12 mg/l et de préférence de 4,4 à 12 mg/l. Pour ces valeurs de C/N la production de caroténoïdes est égale à celle que l'on trouve naturellement dans Haematococcus et est donc faible.

Les inventeurs ont trouvé qu'une quantité relativement importante d'azote dissous était nécessaire pour la production de biomasse. En revanche, une carence en azote du milieu de culture, pour une quantité donnée de biomasse, était favorable à la production de caroténoïdes.

Aussi, la concentration en azote dans le milieu de culture, en fin de cycle cultural, est choisie avantageusement voisine de 0. (Plus la concentration en azote est faible, plus la quantité de caroténoïdes produite est élevée, pour une même quantité de carbone dans le milieu de culture).

Pratiquement la concentration en azote, en fin de culture est égale à 0,1 mg/l, la concentration en carbone pendant la phase de production de caroténoïdes étant maintenue entre 0,22 et 12 mg/l, dans le milieu de culture.

Avantageusement, les composés carbonés sont introduits dans le milieu de culture sous forme de gaz carbonique pur ou mélangé avec de l'air. Toutefois, il est possible de les introduire sous forme carbonates et notamment de carbonates de calcium, de magnésium, etc.

De même, les composés azotés sont introduits avantageusement dans le milieu de culture sous forme d'au moins un composé choisi parmi les sels d'ammonium, les nitrates et l'urée. Comme sels d'ammonium, on peut citer $NH_4Cl$ et comme nitrates on peut citer le nitrate de sodium, de potassium, ...

Le pH du milieu de culture est maintenu à un pH neutre allant de 6,5 à 7,5 et par exemple égal à 7.

Le dioxyde de carbone dissous dans le milieu aqueux, noté $CO_2d$ et responsable de l'apport en carbone des microalgues, est soumis aux équilibres chimiques I suivants :

$$CO_2d \rightleftarrows H_2CO_2 \rightleftarrows HCO_3^- \rightleftarrows CO_3^{--} \qquad (I)$$

qui sont sous la dépendance du pH entre 4 et 12.

L'adjonction de $CO_2$ dans le milieu de culture devrait normalement entraîner une acidification du milieu. Mais étant donné que les microalgues absorbent très rapidement le $CO_2$ (la cinétique de la réaction photosynthétique est au plus égale à 1 seconde), les équilibres chimiques I ont tendance à se déplacer de la droite vers la gauche, ce qui correspond à une alcalinisation du milieu. Autrement dit, si la demande en $CO_2$ des microalgues est plus élevée que l'apport en $CO_2$ dans le milieu, le pH de ce dernier se déplace vers les valeurs supérieures à 7,5.

Pour ramener le pH entre 6,5 et 7,5, on peut soit augmenter l'apport en $CO_2$ et arriver à une stabilisation du pH si la demande et l'apport en $CO_2d$ sont égaux ou bien ajouter au milieu de culture un acide fort tel que HCl, $HNO_3$, $H_3PO_4$. La normalité de l'acide ajouté varie de 2 à 4.

L'adjonction d'acide nitrique modifie, en plus du pH, la quantité d'azote présent dans le milieu de culture, ce qui peut être gênant pour le contrôle en continu de l'apport en azote aux microalgues.

L'acidification du milieu de culture est de préférence réalisé avec l'acide orthophosphorique qui du fait de ces trois constantes de dissociation présente un excellent pouvoir tampon. En outre, l'ion $PO_4^{3-}$ est très apprécié par les microalgues et constitue l'un de leurs ions nécessaires à leur croissance.

En plus de la dépendance de la demande photosynthétique en $CO_2$, le pH du milieu de culture dépend, dans une certaine mesure de l'alimentation en azote comme le montrent les deux équations biologiques suivantes II, III données dans le cas de l'astaxanthine.

$$\text{(II)} \qquad 106CO_2 + 138H_2O + 16NO_3^- \rightarrow \text{biomasse de microalgue} + 1380_2 + 16\ OH^-$$

$$\text{(III)} \qquad 106CO_{2+} + 106H_2O + 16NH_4^+ \rightarrow \text{biomasse de microalgue} + 1060_2 + 16\ H$$

Le déplacement des équilibres I ci-dessus dépend de plus de l'intensité lumineuse de la lumière incidente nécessaire à la croissance des microalgues. La lumière peut être naturelle ou artificielle et son

intensité va de 30 à 400 $W/m^2$ par jour.

Un autre facteur jouant sur les équilibres I ci-dessus est la concentration cellulaire du milieu de culture. Cette concentration cellulaire peut être mesurée par le nombre de cellules par unité de volume (prélèvement d'un échantillon que l'on place dans un microscope), par le poids de matière sèche par unité de volume (ce qui correspond au prélèvement d'un échantillon, au filtrage de l'échantillon, au séchage à 100 °C puis au pesage de la matière obtenue) ou bien par la densité optique du milieu de culture.

La concentration cellulaire d'un milieu de culture estimée par la mesure de la densité optique, notée DO, suit la loi de Beer-Lambert dont on rappelle le principe.

La fonction de lumière LnI/Io absorbée par un échantillon de culture, d'épaisseur e (exprimée en cm), de poids sec PS (exprimé en g/l) à une longueur vaut :

$$DO = LnI/Io = \epsilon(\lambda) x PS x e$$

équation dans laquelle $\epsilon(\lambda)$ représente le coefficient d'extinction moléculaire, à la longueur d'onde $\lambda$, du milieu de culture que l'on étudie ; Io représente l'intensité lumineuse de la lumière incidente ; I représente l'intensité lumineuse en sortie de l'échantillon et Ln représente le logarithme népérien.

On trace alors le spectre d'absorption $\epsilon(x)$ de la culture étudiée et on effectue des mesures à la longueur d'onde correspondant au maximum d'absorption, soit $DO_{max}$ et à la longueur d'onde correspondant au minimum d'absorption, soit $DO_{min}$. Si l'on suppose $\epsilon(x)$ constant pour une culture donnée, alors la densité optique DO permet de suivre l'évolution de la biomasse totale ou du nombre de cellules de microalgues par unité de volume, exprimé en poids sec.

Avantageusement, le gaz carbonique est introduit dans le milieu de culture en mélange avec de l'air ; la quantité d'air utilisée représente de préférence 5 à 10 volumes d'air par volume de culture et par heure.

Pour favoriser la croissance des microalgues et donc la production en caroténoïdes, le milieu de culture est maintenu avantageusement à une température de 20 à 25 °C et de préférence égale à 20 °C.

Selon l'invention, la quantité de carbone et la quantité d'azote présentes dans le milieu de culture sont contrôlées en continu.

A cet effet, la quantité de $CO_2$ dissous dans le milieu de culture est constamment mesurée soit à l'aide d'une sonde à $CO_2$ dissous, soit par mesure du $CO_2$ gazeux à l'entrée et à la sortie du réacteur de culture, cette mesure étant effectuée par spectrométrie infrarouge, soit encore en déterminant la quantité de $CO_2$ réellement consommée par les microalgues en mesurant la quantité de $O_2$ émise par ces dernières. En effet, chaque fois qu'une molécule de $CO_2$ est fixée photosynthétiquement par une microalgue, une molécule de $O_2$ est émise. Le suivi de la concentration en oxygène dissous du milieu de culture, avec une électrode à $O_2$ dissous (Ingold), permet d'effectuer le contrôle en $CO_2$.

Ces différents moyens peuvent être utilisés seuls ou en redondance afin de contrôler parfaitement l'alimentation en carbone des microalgues, le tout étant bien entendu, ramené à l'unité de concentration cellulaire. Cette dernière peut être stabilisée par application du principe de la culture en continu, en utilisant un taux de dilution constant correspondant à un temps de séjour dans le réacteur qui peut aller de deux à 20 jours. Le taux de dilution est lié au taux de croissance des microalgues et correspond à la quantité de milieu nourricier introduit par heure.

Les différents contrôles possibles de l'alimentation çarbonée des microalgues ne sont bien entendu valables que dans la mesure où le pH est maintenu dans une zone allant de 6,5 à 7,5.

Le dispositif selon l'invention s'applique avantageusement à la production l'astaxanthine responsable de la couleur rose de certains crustacés et poissons.

La quantité d'astaxanthine produite est en particulier déterminée par spectrophotométrie, c'est-à-dire par mesure de la densité optique de l'astaxanthine à 478 nm présente dans le milieu de culture ; la relation entre DO et la concentration en astaxanthine est donnée par la loi de Beer-Lambert.

Bien que l'invention s'applique parfaitement bien à la production d'astaxanthine, elle s'applique à d'autres caroténoïdes. En particulier une culture selon l'invention d'Haematococcus pluvialis permet la production de $\beta$ carotène, d'adoirubine, de luteïne, de violaxanthine et de néoxanthine, en plus d'astaxanthine, servant en particulier dans l'agro-industrie et la pharmacologie.

L'invention a donc pour objet un dispositif pour la production de caroténoïdes à partir d'une culture d'Haematococcus pluvialis, caractérisé en ce qu'il comprend :

- un photobioréacteur apte à contenir un milieu de culture,
- des moyens d'alimentation en composé carboné du milieu de culture,
- des moyens de contrôle de la quantité de carbone présente dans le milieu de culture,
- des moyens d'alimentation en composé azoté du milieu de culture, et
- des moyens de contrôle de la quantité d'azote présente dans le milieu de culture.

D'autres caractéristiques et avantages de l'invention ressortiront mieux de la description qui va suivre, donnée à titre illustratif et non limitatif en référence aux figures annexées, dans lesquelles :

- la figure 1 représente schématiquement un dispositif conforme à l'invention,
- les figures 2 et 3 sont des courbes donnant les variations de la densité optique de l'astaxanthine et du milieu de culture au cours du temps, pour différentes valeurs du rapport C/N à respectivement 478 et 760 nm.

Une souche d'Haematococcus pluvialis qui est une microalgue du type des microchlorophycées est cultivée dans un réacteur 2 comportant un photobioréacteur de culture 4 pourvu dans sa partie inférieure d'une conduite d'amenée 6 du substrat nourricier nécessaire à la croissance des microalgues présentes dans le photobioréacteur 4. Cette conduite 6 est équipée d'une pompe 8 de mise en circulation du substrat nourricier 11.

A son sommet, le photobioréacteur 4 est pourvu d'une conduite de sortie 10 du substrat nourricier appauvri, cette dernière débouchant au sommet d'un carbonateur 12 ou réacteur à $CO_2$. Ce carbonateur est formé d'une colonne d'aération en verre ou en plastique. La base de ce carbonateur 12 est connectée à la conduite d'amenée 6.

Les flèches représentées sur la figure 1 indiquent le sens de circulation des différentes substances nécessaires à la croissance des microalgues contenues dans le photobioréacteur 4.

Pour de plus amples détails sur la constitution du carbonateur et du photobioréacteur on peut se référer à l'article de C. Gudin et C. Thepenier cité précédemment.

Le carbonateur 12 est alimenté en son sommet, via une conduite d'amenée 14, en substrat nourricier 11 constitué du milieu de Bristol dont la composition est donnée dans l'article de Amer j. Botany de Starr RC, 1964, n°51, pp. 1013-1044 intitulé "Culture collection of Indiana". En outre cette solution contient le complexe du fer et de l'acide éthylènediaminetétracétique (EDTA).

Une pompe 16 montée sur la conduite d'amenée 14 assure l'injection du milieu de Bristol dans le carbonateur 12.

Une conduite 18 débouchant à la base du carbonateur 12 permet d'alimenter ce dernier en un mélange d'air et de $CO_2$. Ce mélange représente 5 à 10 fois le volume de la culture par heure, et contient de 0,1 à 10% en volume de $CO_2$. Cette conduite 18 est équipée d'un débitmètre 20 permettant de régler le débit d'alimentation en $CO_2$.

La base du carbonateur 12 est pourvue d'un diffuseur 22 ou aérateur, réalisé en verre fritté, permettant la diffusion du mélange gazeux dans le milieu de Bristol 11.

La quantité de $CO_2$ se dissolvant dans le milieu nourricier 11 dépend de la forme et des dimensions du carbonateur 12 et en particulier de son rapport surface/volume ainsi que de la forme du diffuseur 22, et notamment de sa porosité, et de sa surface.

L'étape de la dissolution du $CO_2$ dans le substrat 11 (correspondant au passage de la phase gaz à la phase liquide) est la plus longue du procédé et correspond à l'hydratation de l'ion $CO_2$ qui est de l'ordre de 26,6 secondes à 25°C ; les autres étapes du procédé sont voisines de $10^{-15}$ secondes, hormis la consommation en $CO_2$ dissous par les microalgues contenues dans le photoréacteur 4 qui est au plus égale à 1 seconde.

Le réacteur 2 est placé en lumière naturelle ou articielle et un détecteur 24 situé à proximité du photobioréacteur 4, du type pyranomètre de Eppley, permet de mesurer l'intensité lumineuse à laquelle sont soumises les microalgues contenues dans le photoréacteur 4. Le détecteur 24 est relié à un ordinateur 30, tel que celui vendu par la société Hewleh Packard sous la référence HP85, auquel il fournit ladite mesure.

Le photobioréacteur 4 est en outre logé dans un bain thermostaté 26 dans laquelle circule de l'eau qui peut être chauffée ou refroidie à volonté. L'alimentation en eau 27 débouchant à la base de l'enceinte 26 est équipée d'une électrovanne 28 dont la commande est assurée par l'ordinateur 30, en fonction de la température de l'eau mesurée à l'aide d'une sonde 32 plongeant dans l'enceinte 26 et fournissant le signal de mesure à l'ordinateur 30.

Le système de chauffage et de refroidissement de l'eau de circulation, bien connu de l'homme de l'art, n'est pas représenté sur la figure 1 et se situe à l'extérieur du réacteur 2.

Le pH de la solution ayant tendance à s'alcaliniser du fait de la consommation en $CO_2$ par les microalgues, une conduite d'amenée 34 en acide orthophosphorique, débouchant au sommet du carbonateur 12 doit être prévue. Cette conduite 34 est équipée d'une électrovanne 36 dont l'ouverture et la fermeture sont asservies, via l'ordinateur 30, à la mesure du pH du milieu de culture effectuée par une sonde 38 plongeant dans le photobioréacteur 4.

La conduite d'amenée 18 est pourvue d'une déviation 40, permettant un prélèvement du mélange injecté dans le carbonateur 12, sur laquelle est monté un analyseur à infra-rouge 42 pour mesurer la

quantité de $CO_2$ gazeux présent à l'entrée du carbonateur 12, cet analyseur étant relié à l'ordinateur 30.

De même, une sortie 44 des gaz introduits dans le carbonateur 12 est prévue au sommet de ce dernier. Cette conduite 44 est équipée d'un analyseur à infrarouge 46 relié aussi à l'ordinateur 30, servant à mesurer la quantité de $CO_2$ non dissous dans le milieu de culture. Par comparaison des signaux fournis par les analyseurs 42 et 46 il est possible de connaître la quantité de $CO_2$ dissous dans le milieu de culture et qui par conséquent peut être photosynthétisé par les microalgues contenues dans le photobioréacteur 4.

Le contrôle en azote du milieu de culture, est assuré à l'aide d'une électrovanne 48, montée sur la conduite d'amenée 14 du substrat nourricier dont la fermeture et l'ouverture sont commandées par l'ordinateur 30. L'ouverture et la fermeture de cette vanne 48 fixent le débit du substrat nourricier frais, c'est-à-dire le taux de dilution du milieu de culture pour une production en continu de caroténoïdes.

La quantité d'azote présente dans le milieu de culture est régulièrement mesurée (2 fois par jour) par prélèvement d'un échantillon du milieu de culture que l'on introduit dans une chaîne de mesure à azote, connue, telle que l'analyseur à azote de chez Technicon®.

L'extraction des microalgues enrichies en caroténoïdes et notamment en astaxanthine est effectuée de façon connue, par centrifugation, ultrafiltration ou comme décrit dans l'article de B. Renström et al paru dans Phytochemistry vol.20, n°11, pp.2561-2564, intitulé "Optical purity of Astaxanthin from Haematococcus pluvialis".

Les microalgues sont reçues dans un récipient 50 relié au milieu du carbonateur via une conduite d'amenée 52.

EXEMPLE

a) croissance d'*Haematococcus pluvialis*

Avec comme substrat nourricier en début de culture un milieu de Bristol de composition suivante :

$NaNo_3$ 0,25 g/l
$CaCl_2, 2H_2O$ 0,025 g/l
$MgSO_4, 7H_2O$ 0,075 g/l
$K_2HPO_4$ 0,075 g/l
$KH_2PO_4$ 1,75 g/l
NaCl 0,025 g/l

un pH de 7, une température de 20°C, un éclairage artificiel continu de 30 W/m$^2$ par jour, 5 volumes d'air par volume de culture et par heure contenant 5% de $CO_2$, il faut environ 20 jours pour atteindre la phase stationnaire de croissance d'Haematococcus pluvialis ; la croissance exponentielle des microalgues correspond à l'équation biochimique IV de Myers suivante :

$$\text{(IV)} \quad 6{,}14CO_2 + 3{,}65H_2O + NH_3 \rightarrow C_{6,14}H_{10,3}O_{22}N + 6{,}820_2$$

dans laquelle la biomasse de microalgue est verte.

Le rapport C/N dans le milieu de culture, ci-dessus, en début de cycle est égal à 0,3.

La concentration cellulaire est déterminée par spectrophotométrie, c'est-à-dire en fonction de la densité optique de la culture, à 760 nm correspondant au maximum d'absorption d'*Haematococcus pluvialis*.

b) production d'astaxanthine.

Au bout de 20 jours, les microalgues se pigmentent en rouge grâce à un caroténoïde, identifié comme l'astaxanthine. La concentration en astaxanthine est mesurée par la densité optique de la culture, à 478 nm, qui correspond au maximum d'absorption de l'astaxanthine.

Le maximum de pigmentation des microalgues donc de la concentration en astaxanthine ainsi que des autres caroténoïdes synthétisés par *Haematococcus pluvialis* se situe dans la phase stationnaire de croissance des microalgues correspondant à une carence en azote dans le milieu de culture, et plus le milieu de culture est concentré en microalgues, plus forte est la concentration en astaxanthine (et en d'autres carotéoïdes).

Le fait d'augmenter la concentration initiale d'azote dans le milieu de culture permet d'augmenter de façon importante la production d'astaxanthine par les microalgues, comme ceci ressort clairement des figures 2 et 3.

La figure 2 donne les variations de la densité optique à 478 nm exprimée en unité arbitraire, et donc les variations de la concentration en astaxanthine, en fonction du temps exprimé en jour. Les conditions

opératoires sont celles données au paragraphes a avec une concentration en $NaNO_3$ dans le milieu de Bristol qui varie, le pourcentage de $CO_2$ dans les 5 volumes d'air étant égal à 0,1.

La courbe A correspond à un milieu de culture initial contenant 0,25 g/l de $NaNO_3$, la courbe B à une quantité initiale de 0,5 g/l de $NaNO_3$ et la courbe C à une concentration initiale de 0,75 g/l de $NaNO_3$.

En début de cycle cultural, la courbe A correspond à un rapport C/N de 0,0055, la courbe B à un rapport C/N de 0,00275 et la courbe C à un rapport C/N de 0,0018. Au bout de 24 jours soit en fin de culture, la densité optique du milieu de culture n'a fait que doubler pour la courbe A, alors qu'elle a triplé pour la courbe B et quadruplé pour la courbe C, ce qui correspond respectivement à un doublement, triplement et quadruplement de la concentration en astaxanthine.

En fin de cycle, le rapport C/N pour les trois courbes vaut 2,2 environ.

Le fait de doubler la dose, voire tripler la dose initiale en nitrate et donc en azote du milieu de culture, conduit à une augmentation importante de la production en astaxanthine par augmentation de la croissance ou développement des microalgues.

On donne ci-après le calcul permettant de trouver le rapport C/N dans le milieu de culture. x g/l de $NaNO_3$ correspond à 14.x/85 g/l d'azote et y% de $CO_2$ gazeux dans le mélange air-$CO_2$ correspond à 40.y/5 mg/l de $CO_2$ dissous (mesuré avec des analyseurs à infrarouge) et donc à 12.y/5 mg/l.

Il est aussi possible d'augmenter la production en astaxanthine par Haematococcus pluvialis en maintenant constante la quantité d'azote dissoute dans le milieu de culture et en augmentant la quantité de carbone dissoute dans ce milieu. Ceci apparaît clairement sur la figure 3 qui donne les variations de la densité optique à 478 nm, notée $D.O_{478}$, donc celles de la concentration en astaxanthine, et les variations de la densité optique à 760 nm, notée $D.O_{760}$, donc celles de la concentration en microalgues, en fonction du temps.

Les conditions de culture sont les mêmes que celles données en a avec une quantité de $CO_2$ injectée dans la culture variable.

Les courbes E, I et G correspondent à une injection, dans le milieu de culture, de 5 volumes d'air par volume de culture et par heure, enrichi à 1% de $CO_2$ et les courbes F, J et H, à un air enrichi à 2% de $CO_2$. Les courbes E et F donnent la densité optique à 478 nm et correspondent respectivement à un rapport C/N en début de cycle cultural de 0,055 et de 0,11 et en fin de cycle cultural de 2,2. On constate d'après ces courbes que la densité optique et donc la concentration en astaxanthine croît avec le rapport C/N, c'est-à-dire quand C croît.

Les courbes des figures 2 et 3 ont été effectuées en culture discontinue ou système Batch par ajustement de la concentration cellulaire initiale à une valeur de densité optique de l'ordre de 0,5, à 478 nm. Cette culture a été stoppée au bout de 24 jours, après consommation totale de l'azote et donc des nitrates dissous dans le milieu de culture. Ceci ressort clairement des courbes G et H de la figure 3 qui sont des courbes de disparition de l'azote dans le milieu de culture, mesurées par la diminution de la densité optique DO du milieu à 220 nm.

Les courbes I et J de la figure 3 donnent la densité optique à 760 nm pour respectivement un rapport C/N de 0,055 et de 0,11 en début de culture. Le fait d'augmenter la quantité de C pour une valeur de N fixe conduit à augmenter la concentration cellulaire, et donc la concentration en astaxanthine.

Les figures 2 et 3 ont été établies avec un spectrophotomètre à double faisceau et à réponse linéaire entre 0 et 3 commercialisé sous la marque UVICON. Le calibrage des cuves de mesure et de référence a été effectuée à 478 nm avec le milieu de Bristol donné en a, ce milieu étant ensuite utilisé comme solution de référence pour déterminer la concentration en microalgues et la concentration en astaxanthine.

Les cuves utilisées avaient une épaisseur e de 1 cm. Le coefficient d'extinction moléculaire d'Haematococcus pluvialis à 760 nm vaut 3 environ, celui de l'astaxanthine n'est pas encore connu avec certitude.

D'où la concentration en Haematococcus pluvialis PS est régie par l'équation $PS = D.O_{760} x 3,3$. Aussi, pour $D.O_{760} = 0,8$ donnée sur la courbe J de la figure 3, PS vaut 2,6 g/l.

Pour la production en continu d'astaxanthine, la concentration en carbone dissous est choisie constante pendant toute la durée de production ; cette concentration est notamment égale à 12 mg/l (soit 5% de $CO_2$ en mélange avec 5 volumes d'air par volume de culture) et son introduction dans le milieu de culture se fait en continu.

Pour la quantité d'azote dissous, on choisit une alimentation discontinues après chaque période d'introduction du milieu de Bristol dans le milieu de culture, la concentration en azote dissous dans la culture est notamment de 120 mg/l (soit 0,75 g/l de $NaNO_3$) et avant chaque période d'introduction du milieu de Bristol, la concentration en azote dissous dans la culture est égale à 0,1 mg/l. La période de renouvellement du milieu de Bristol (ou taux de dilution du milieu de culture) est de 20 jours.

La description précédente a permis de montrer le rôle clé du rapport C/N dans le milieu de culture sur la production d'astaxanthine à partir d'Haematococcus pluvialis. A ce jour, aucune publication relative à

cette souche de microalgues ne mentionne le rôle joué par ce rapport sur la pigmentation de la microalgue.

## Revendications

1. Dispositif pour la production de caroténoïdes à partir d'une culture d'Haematococcus pluvialis, caractérisé en ce qu'il comprend :
   - un photobioréacteur (4) apte à contenir un milieu de culture,
   - des moyens d'alimentation (18, 12, 6) en composé carboné du milieu de culture,
   - des moyens de contrôle (20, 18, 42, 46, 30) de la quantité de carbone présente dans le milieu de culture,
   - des moyens d'alimentation (14, 11) en composé azoté du milieu de culture, et
   - des moyens de contrôle (48, 30) de la quantité d'azote présente dans le milieu de culture.

2. Dispositif selon la revendication 1, caractérisé en ce que des moyens (30, 36, 38) de contrôle du pH du milieu de culture sont prévus.

3. Dispositif selon la revendication 1 ou 2, caractérisé en ce que des moyens (28, 30, 32) de contrôle de la température du milieu de culture sont prévus.

4. Dispositif selon l'une quelconque des revendications 1 à 3, caractérisé en ce que des moyens (24, 30) de contrôle de l'intensité lumineuse reçue par le milieu de culture sont prévus.

## Claims

1. Apparatus for performing the production of carotenoids from a culture of Haematococcus pluvialis, characterized in that it comprises a photobioreactor (4) able to contain the culture medium, means (18, 12, 6) for supplying the culture medium with the carbon-containing compound, means (20, 18, 42, 46, 30) for checking the carbon quantity present in the culture medium, means (14, 11) for supplying the culture medium with the nitrogen-containing compound and means (48, 30) for checking the nitrogen quantity present in the culture medium.

2. Apparatus according to claim 1, characterized in that means (30, 36, 38) for checking the pH of the culture medium are provided.

3. Apparatus according to claims 1 or 2, characterized in that the means (28, 30, 32) for checking the temperature of the culture medium are provided.

4. Apparatus according to any one of the claims 1 to 3, characterized in that means (24, 30) for checking the light intensity received by the culture medium are provided.

## Patentansprüche

1. Vorrichtung zur Herstellung von Karthinoiden, ausgehend von einer Kultur von Haematococcus pluvialis, dadurch **gekennzeichnet,**
   daß sie:
   - einen Photobioreaktor (4), der fähig ist, ein Milieu für die Kultur in sich zu schließen,
   - Hilfsmittel zur Versorgung (18, 12, 6) des Kulturmilieus mit kohlenstoffhaltigen Verbindungen,
   - Hilfsmittel zur Kontrolle (20, 18, 42, 46, 30) der Kohlenstoffmenge, die in dem Kulturmilieu anwesend ist,
   - Hilfsmittel zur Versorgung (14, 11) des Kulturmilieus mit Stickstoffverbindungen, und
   - Hilfmittel zur Kontrolle (48, 30) der Stickstoffmenge, die in dem Kulturmilieu anwesend ist, umfaßt.

2. Vorrichtung gemäß Anspruch 1, dadurch gekennzeichnet, daß Hilfsmittel (30, 36, 38) zur Kontrolle des pH-Werts des Kulturmilieus vorgesehen sind.

3. Vorrichtung gemäß den Ansprüchen 1 oder 2, dadurch gekennzeichnet, daß Hilfsmittel (28, 30, 32) zur Kontrolle der Temperatur des Kulturmilieus vorgesehen sind.

4. Vorrichtung gemäß einem der Ansprüche 1 bis 3, dadurch gekennzeichnet, daß Hilfsmittel (24, 30) zur Kontrolle der vom Kulturmilieu empfangenen Lichtintensität vorgesehen sind.

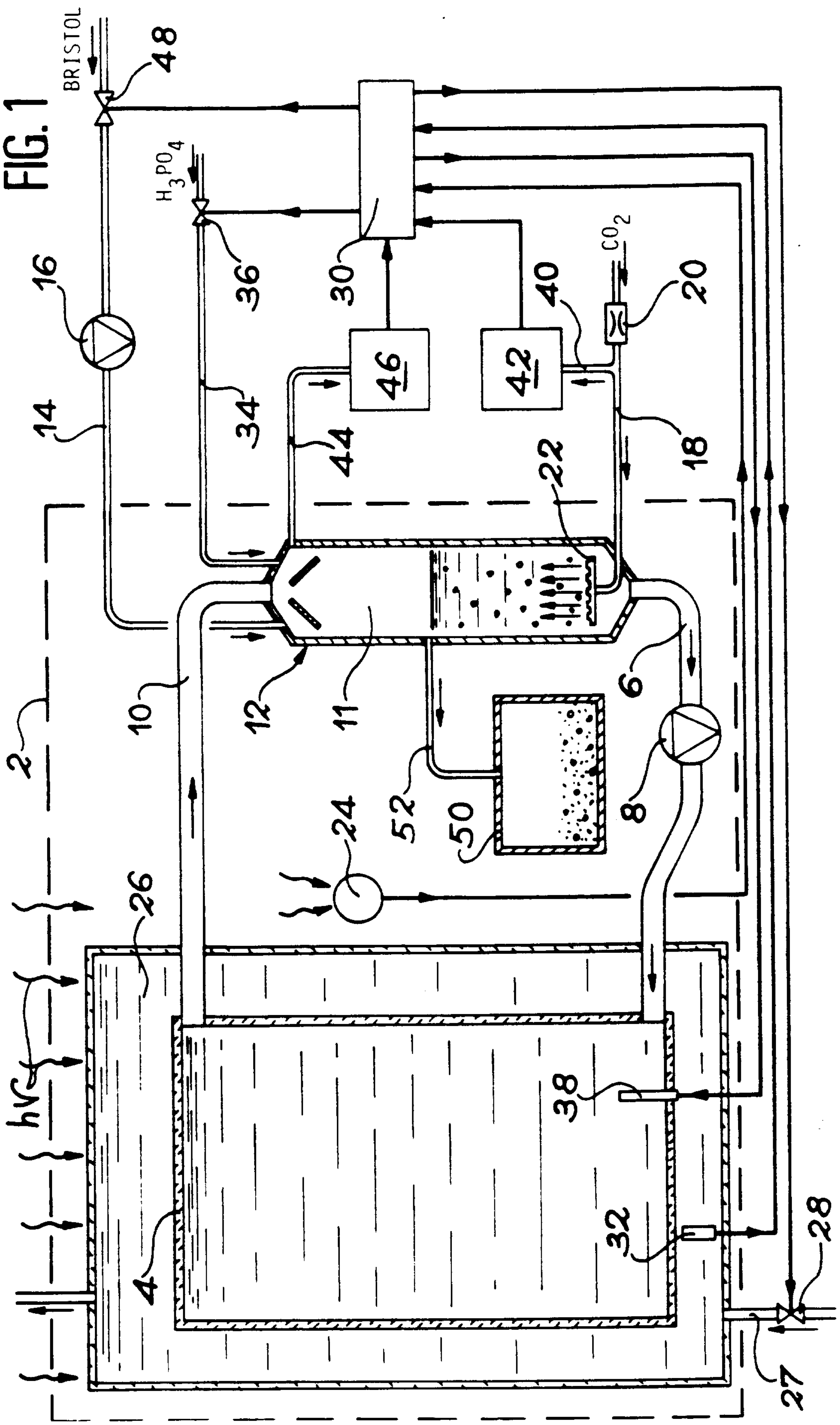
BRISTOL
$H_3PO_4$
$CO_2$
hV
48
16
14
36
34
30
46
42
40
20
18
44
22
2
10
12
11
6
24
52
50
8
26
38
4
32
28
27

FIG. 1

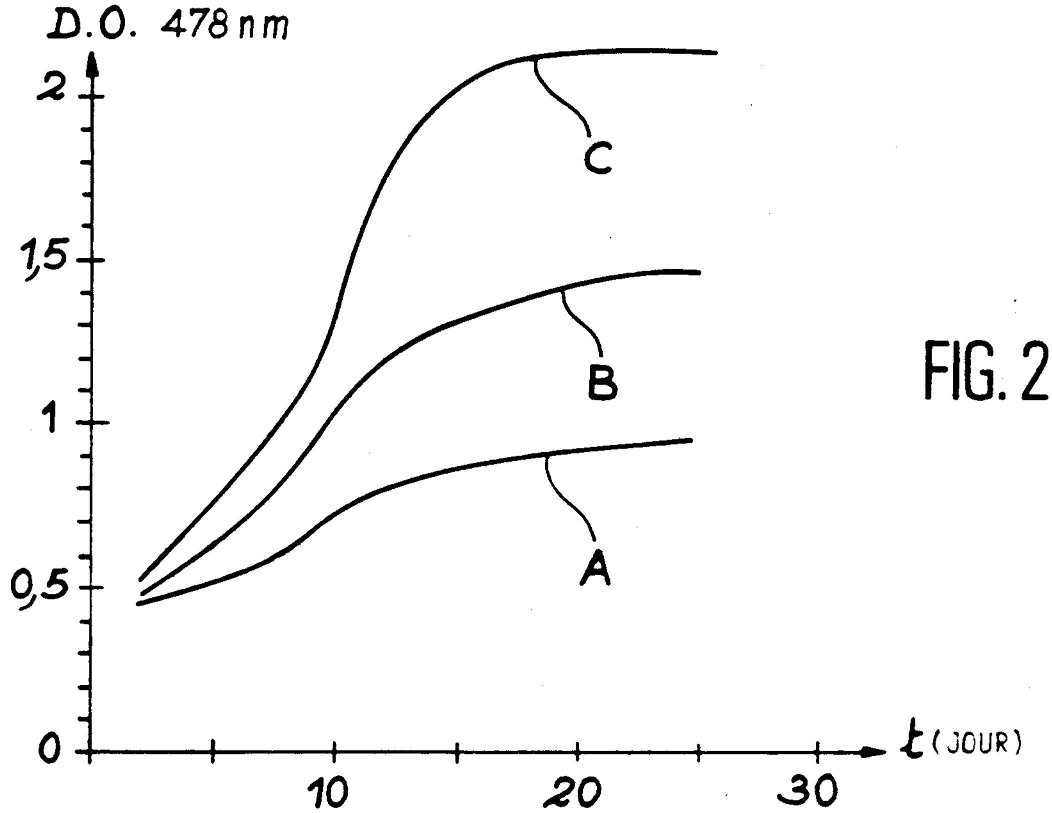
D.O. 478 nm
2
1,5
1
0,5
0
C
B
A
t (JOUR)
10
20
30

FIG. 2

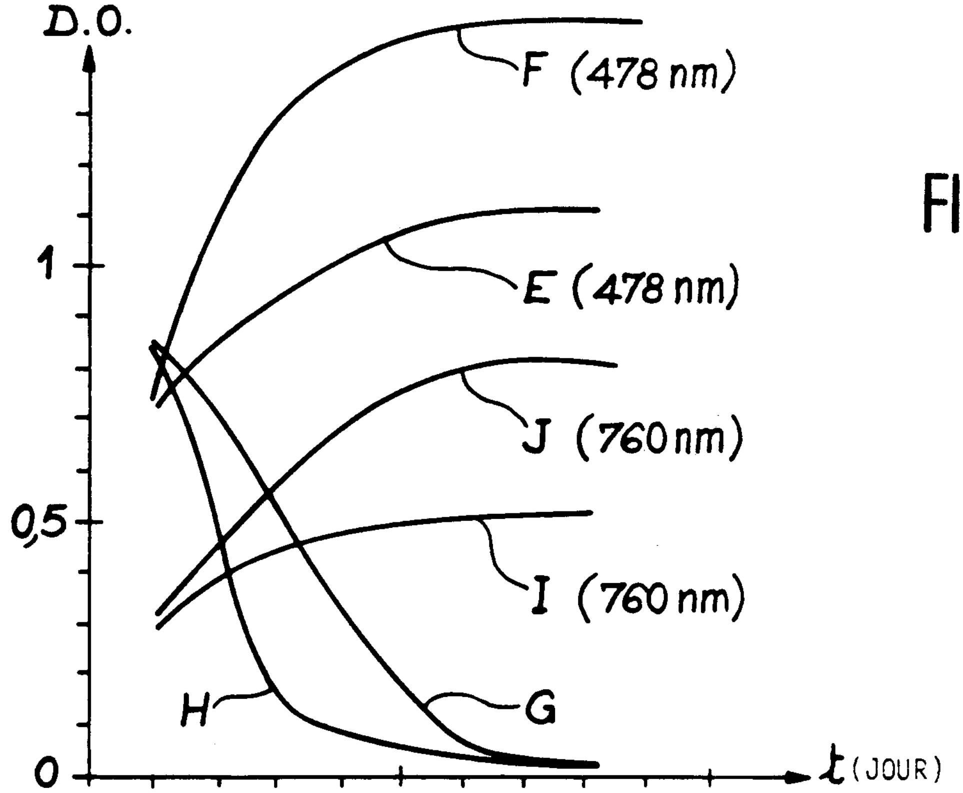
D.O.
1
0,5
0
F (478 nm)
E (478 nm)
J (760 nm)
I (760 nm)
H
G
t (JOUR)

FIG. 3